# EUROPEAN PATENT APPLICATION

(11) **EP 1 997 911 A2**
(43) Date of publication of application: **03.12.2008**
(21) Application number: 08009943.5
(22) Date of filing: 30.05.2008
(51) Int. Cl.: C12Q 1/68

(54) **Method for detecting oral squamous-cell carcinoma and method for suppressing the same**

(30) Priority: 30.05.2007 JP 2007143110
(71) Applicant: FUJIFILM Corporation, Minato-ku Tokyo (JP); Tokyo Medical and Dental University, Tokyo 113-8510 (JP)
(72) Inventor: Inazawa, Johji, Tokyo 113-8510 (JP); Imoto, Issei, Tokyo 113-8510 (JP); Suzuki, Emina, Tokyo 113-8510 (JP)
(74) Representative: HOFFMANN EITLE

(57) **Abstract**

An object of the present invention is to provide a method for detecting cancer through identification of genes exhibiting characteristic behavior in the cases of cancer such as oral squamous-cell carcinoma, and a cell growth inhibitor. The present invention provides a method for detecting cancer, which comprises detecting canceration including malignancy of a specimen through detection of at least one alteration of a gene existing in a chromosomal region 1q21, 2q24.1-q24.2, 3p13, 7p11.2, 10p12.1, 11p15.4, 11p15.2, 11p13.3, 11q22, 11q23.3, 12p13, 12q24.31, 13q33.3-q34, 12q24.1, 19q13, or 22q12.1 in the specimen.

## Description

### Technical Field

The present invention relates to a method for detecting cancer by detecting gene alterations that exist in specific chromosomal regions for the purpose of early diagnosis of oral squamous-cell carcinoma through observation of the genotype thereof.

### Background Art

Oral squamous-cell carcinoma (OSCC) is classified as a head and neck cancer and is a tumor that is mainly generated from oral mucous membrane epithelia and the like. Among head and neck cancers, OSCC incidence is as high as about 35% and has some influences on 270,000 people worldwide every year (Parkin, DM., et al., CA Cancer J Clin. 55, 74-108, 2002). The most common site of the origin of oral squamous-cell carcinoma is tongue, and the second most common site thereof is gingiva (gum). Oral squamous-cell carcinoma is known to be developed at other mucous membranes of the oral cavity such as buccal mucosa, palate, and mouth floor. Furthermore, oral squamous-cell carcinoma is also known to be developed at jaw bone or salivary gland.

In recent years, although procedures for diagnosis and treatment for oral squamous-cell carcinoma have been advanced, the prognosis thereof has remained unimproved. Accordingly, it has been desired to discover a causative gene for oral squamous-cell carcinoma and to elucidate the functions for establishment of new strategic understandings for effective therapeutic methods and chemical prevention.

### Disclosure of the Invention

Successful elucidation of the mechanism of canceration of oral-cavity-derived cells and mainly oral-epithelium-derived cells at the gene level will enable detection of canceration of oral-cavity-derived cells at the gene level, diagnosis of the malignancy of oral squamous-cell carcinoma, and suppression of the advancement thereof.
Furthermore, it will also enable establishment of methods for selecting and developing drugs, as well as therapeutic methods based on such mechanisms. Specifically, this object can be achieved by identifying genes exhibiting characteristic behavior observed in oral squamous-cell carcinoma cases and then carrying out technical examination mainly targeting such genes. Hence, an object to be achieved by the present invention is to provide a method for detecting cancer through identification of genes exhibiting characteristic behavior in the cases of cancer such as oral squamous-cell carcinoma, and a cell growth inhibitor.

Comparative Genomic Hybridization (CGH) is the best method for conveniently and rapidly analyzing genetic abnormalities accompanying amplification or deletion of numerous genes in the genome or inactivation of genes. To analyze genetic abnormalities in the genome involved in canceration and higher cancer malignancy, the present inventors have selected 800 types or 4500 types of BAC/PAC DNA to be subjected to CGH assay (MCG CancerArray-800, MCG Whole Genome-4500; Takada H., et al., Cancer Sci. 96, 100-105, 2005, Inazawa J., et al., Cancer Sci. 95, 559-563, 2004). As a result, the present inventors have succeeded in identification of a cancer-associated gene that promotes canceration of oral-cavity-derived cells; that is, a Phosphoribosyl transferase domain containing 1 (PRTFDC1) gene. Moreover, the present inventors have succeeded in discovering that deletion or inactivation of the PRTFDC1 gene, and specifically a decrease in the PRTFDC1 protein, significantly promote the proliferation of oral squamous-cell carcinoma and that the increased level of a transcript or the protein of the PRTFDC 1 gene results in significantly decreased levels of squamous-cell carcinoma proliferation. Thus, the present inventors have completed the present invention.

Thus, the present invention a method for detecting cancer, which comprises detecting canceration including malignancy of a specimen through detection of at least one alteration of a gene existing in a chromosomal region 1q21, 2q24.1-q24.2, 3p13, 7p11.2, 10p12.1, 11p15.4, 11p15.2, 11p13.3, 11q22, 11q23.3, 12p13, 12q24.31, 13q33.3-q34, 12q24.1, 19q13, or 22q12.1 in the specimen.

Preferably, the gene is at least one of BCL9, MITF, EGFR, PTH, BCL1, FGF4, CCND1, FGF3, PGR, YAP1, CIAP1, MMP7, MMP1, CCND2, FGF6, BCL7A, DP1, GAS6, SUPT5H, PRTFDC1, c10orf63, THNSL1, GPR158, MY03A, or GAF2.

Preferably, the gene alteration is at least one of amplification, deletion, and inactivation.

Preferably, the gene alteration is inactivation due to methylation of a CpG island.

Preferably, The method for detecting cancer of the present invention comprises detecting canceration including malignancy of a specimen through detection of amplification of BCL9, MITF, EGFR, PTH, BCL1, FGF4, CCND1, FGF3, PGR, YAP1, CIAP1, MMP7, MMP1, CCND2, FGF6, BCL7A, DP1, GAS6, or SUPT5H gene or through detection of deletion of PRTFDC1, c10orf63, THNSL1, GPR158, MYO3A, or GAF2 gene.

Preferably, canceration including malignancy of a specimen is detected through detection of deletion or inactivation of a PRTFDC1 gene.

Preferably, the specimen is tissue derived from the oral cavity.

Preferably, the cancer is oral squamous-cell carcinoma.

Preferably, the gene alteration is detected using a DNA chip method, a Southern blot method, a Northern blot method, a real-time RT-PCR method, a FISH method, a CGH method, an array CGH method, a bisulfite sequencing method, or a COBRA method.

The present invention further provides a method for inhibiting cell growth, which comprises introducing a PRTFDC1, c10orf63, THNSL1, GPR158, MY03A, or GAF2 gene, or a protein that is an expression product thereof into cells *in vitro.*

The present invention further provides a cell growth inhibitor, which comprises a PRTFDC1, c10orf63, THNSL1, GPR158, MY03A, or GAF2 gene, or a protein that is an expression product thereof.

The present invention further provides a method for activating cell growth, which comprises introducing siRNA, shRNA, an antisense oligonucleotide, or a loss-of-function type gene of a PRTFDC1, c10orf63, THNSL1, GPR158, MY03A, or GAF2 gene into tumor cells *in vitro.*

The present invention further provides a cell growth activating agent, which comprises siRNA, shRNA, an antisense oligonucleotide, or a loss-of-function type gene of a PRTFDC1, c10orf63, THNSL1, GPR158, MY03A, or GAF2 gene.

The present invention further provides a method for screening for a substance, which comprises causing a test substance to come into contact with oral squamous-cell carcinoma in which gene expression is suppressed due to methylation of a CpG island of a PRTFDC1, c10orf63, THNSL1, GPR158, MYO3A, or GAF2 gene, detecting the expression of the gene, and then selecting a test substance as an anti-tumor substance capable of activating the relevant gene by demethylation of the CpG island of the gene when the gene expression is increased to a level higher than that in a system in which the test substance is not caused to come into contact therewith.

### Brief Description of the Drawings

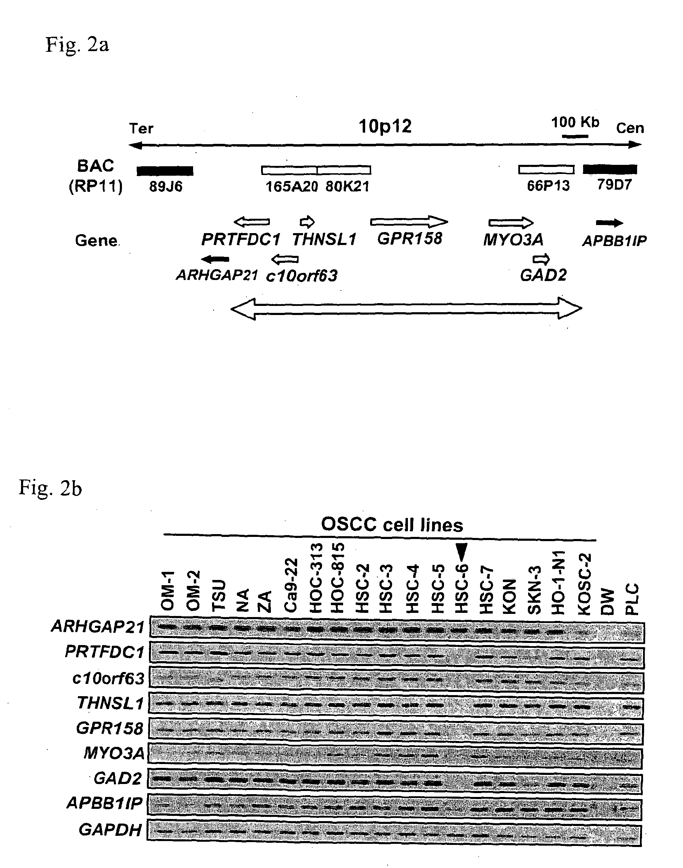

Fig. 1 :
   (a) Frequency of amplification and deletion of copy number in the entire genomes, which was determined using MCG Cancer Array-800 in 18 types of oral squamous-cell carcinoma cell line. Clones were aligned in order of chromosomes 1-22, X, and Y based on the UCSC mapping positions (http://genome. ucsc. edu/ [version May, 2004]. Each asterisk represents a region in which high-level amplification (log2ratio > -2) or homozygous deletion (log2ratio < -2) was observed.
   (b) Typical result obtained by CGH array analysis conducted for the HSC-6 cell line. Significant decreases (log2ratio < -2) in copy numbers of RP11-165A20, 80K21, and 66P13 (arrow) in chromosome 10p12 are shown.
   (c) The result of copy number profiling of chromosome 10 in the HSC-6 cell line. Each arrow indicates a candidate clone in chromosomal region 10p12.
Fig.2 :
   (a) Map that covers a homozygous deletion region of chromosome 10p12 in the HSC-6 cell line. A horizontal line represents BAC spotted on an array (white line: BAC with log2ratio < -2, and black line: BAC with log2ratio> -2). The minimum region of homozygous deletion in the HSC-6 cell line was determined by genomic PCR and is represented by white arrows on both ends. Eight (8) genes located in the periphery of the homozygous deletion region (determined by genomic PCR) in the HSC-6 cell line, homozygous deletions (6 genes), or the remaining genes (2 genes) are represented by white or black lines.
   (b) The results of genomic PCR performed for 8 genes located in the periphery of the homozygous deletion region of chromosome 10p12 and GAPDH gene (control) using 18 types of oral squamous-cell carcinoma cell line.
   (c) The expression of PRTFDC1, c10orf63, THNSL1, GPR158, MY03A, and GAD2 genes in the oral squamous-cell carcinoma cell lines, normal oral epithelium cell line RT7, and normal oral epithelium primary cells was determined by RT-PCR. DW indicates distilled water. An arrowhead indicates the homozygous deletion cell line (HSC-6) shown in Fig. 2b. Among 18 types of cell line, 8 types (OM-1, OM-2, NA, ZA, Ca9-22, HSC-2, HSC-3, and KON) that did not undergo homozygous deletion of the PRTFDC1 gene exhibited complete gene silencing, and one type of the cell line (HOC-313) exhibited decreased expression levels compared with that of a normal control.
   (d) Oral squamous-cell carcinoma cells (NA, HSC-2, and ZA) were treated (+) or not treated (-) with 5-aza-dCyd (5 or 10 µM) for 5 days or with TSA (100 mg) for 24 hours. The results of RT-PCR analysis on the PRTFDC1 gene in the oral squamous-cell carcinoma cells are shown.
Fig.3 :
   (a) Map of a 801-bp CpG island (from -373 to +418) in the periphery of exon 1 in the PRTFDC1 gene. A vertical line represents the CpG site. An open box represents exon 1. The transcription initiation site is indicated with "+1." Thick black lines represent fragments (Fragments 1, 2, and 3) used for promoter assay. Open bars represent regions confirmed by COBRA and bisulfite sequencing (Region 1 and Region 2). Black or gray arrowheads represent restriction enzyme sites *Mlu* I and *Taq* I for the COBRA method.
   (b) Promoter activity of the PRTFDC1 CpG island. A pGL3 empty vector (mock) and reporters containing different sequences of CpG island Regions 1-3 were constructed. NA and HSC-2 cells were transfected with them together with an internal control vector (pRL-hTK). Luciferase activity was normalized with respect to the control. Data indicate average ±SD of values obtained via three independent experiments.
   (c) Typical results of COBRA performed for oral squamous-cell carcinoma cell lines, in which PRTFDC 1 Region 1 and Region 2 were each digested with *Mlu* I and *Taq* I. An arrow indicates a fragment cleaved at the methylated CpG recognition site. An arrowhead indicates (unmethylated) fragment that remained uncleaved. Asterisks represent 8 cell lines in which cleaved DNA fragments were found to be positive.
   (d) The results of bisulfite sequencing of PRTFDC1 Region 1 (62CpG site) and Region 2 (33CpG site), which were confirmed in cells expressing PRTFDC1 and cell lines not expressing PRTFDC1. White and black squares represent an unmethylated CpG site and a methylated CpG site, respectively. Each column represents the one derived from a single type of clone. "NT" means "not tested."
Fig.4 :
   (a) Typical results of COBRA of PRTFDC1 Region 1 and Region 2 in 12 out of 47 cases of oral squamous-cell carcinoma clinical specimen tissue, a positive (HSC-2) control cell line and a negative (HO1-N1) control cell line. An asterisk represents primary oral squamous-cell carcinoma for which the cleaved DNA fragment (arrow) was confirmed.
   (b) The methylation status of PRTFDC1 Region 1 and Region 2 in 4 types of primary cell of oral squamous-cell carcinoma, as determined by bisulfite sequencing.

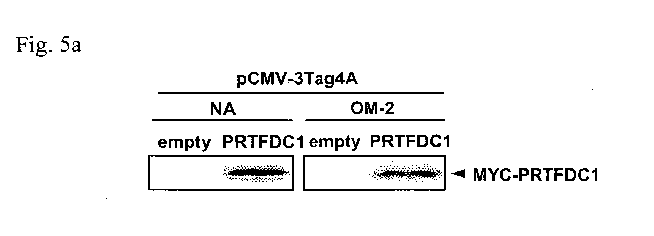

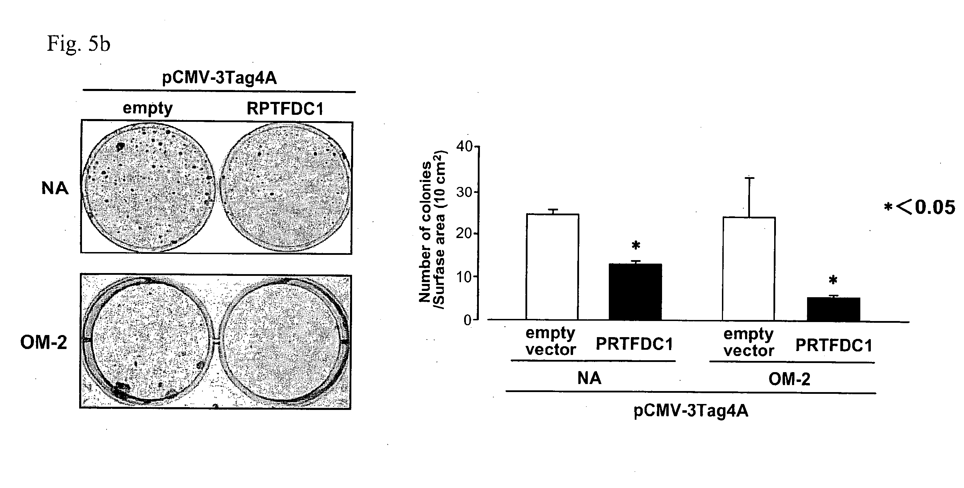

Fig.5 : Effects of PRTFDC1 expression on the proliferation of oral squamous-cell carcinoma. Cell lines (NA and OM-2) lacking PRTFDC1 gene were transfected with pCMV-3Tag4A-PRTFDC1 or pCMV-3Tag4A-mock.
   (a) Western blot analysis was performed using 5 µg of protein extracted from cells at 48 hours after transfection and using an anti-Myc antibody.
   (b) Colony formation assay using cell lines (NA and OM-2) not expressing the PRTFDC1 gene. These cells were transiently transfected with a Myc tag vector (pCMV-3Tag4A-PRTFDC1) containing PRTFDC1 or an empty vector (pCMV-3Tag4A-mock), followed by 3 weeks of drug selection in the presence of G418.
Left: Drug resistant colonies were formed within 3 weeks after transfection, revealing that the degree of colony formation by cells transfected with the PRTFDC1 gene was less than that by cells transfected with the empty vector.
Right: Quantitative analysis of colony formation. Colonies (> 2 mm) were counted. Counting was performed in 3 separate experiments. The result is represented by the average value ±SD of three separate results.

### Best Mode for Carrying Out the Invention

Hereafter, the present invention will be described in detail.

### (1) Method for detecting cancer

The method for detecting cancer according to the present invention comprises detecting canceration including the malignancy of a specimen, through detection of at least one alteration of a gene existing in chromosomal region 1q21, 2q24. 1-q24. 2, 3p13, 7p11. 2, 10p12. 1, 11p15. 4, 11p15. 2, 11p13. 3, 11q22, 11q23. 3, 12p13, 12q24. 31, 13q33. 3-q34, 12q24. 1, 19q13, or 22q12. 1 in the specimen. More specifically, canceration including the malignancy of the specimen can be detected through detection of amplification of BCL9, MITF, EGFR, PTH, BCL1, FGF4, CCND1, FGF3, PGR, YAP1, CIAP1, MMP7, MMP1, CCND2, FGF6, BCL7A, DP1, GAS6, or SUPT5H gene or deletion of PRTFDC1, c10orf63, THNSL1, GPR158, MY03A, or GAF2 gene in a specimen. Particularly preferably, canceration including the malignancy of oral-cavity-derived cells can be detected through detection of deletion or inactivation of PRTFDC1 gene in oral-cavity-derived cells.

As a result of the human genome project, transcripts of the PRTFDC 1 gene are already known. The gene is located on chromosomal region 10p12. Protein encoded by the PRTFDC 1 gene is known to contain a phosphoribosyltransferase domain but the detailed functions thereof remains unknown. The fact that the PRTFDC 1 gene is an important cancer-associated gene involved in the onset of oral squamous-cell carcinoma or the malignancy thereof was unknown before the present invention.

As described above, the detection method comprises detecting deletion or inactivation of PRTFDC1 gene in oral-cavity-derived cells or oral squamous-cell carcinoma.

Preferred examples of oral-cavity-derived cells or oral squamous-cell carcinoma to be subjected to detection of deletion or inactivation of the PRTFDC1 gene are biopsied tissue cells of specimen donors.

Such tissue cell specimen may be a oral-cavity-derived cell of a healthy subject or a cancerous tissue of an oral squamous-cell carcinoma patient. In practice, examples of a major target tissue specimen that can be used herein include: a tissue obtained from a lesion in which suspected canceration of the mucous membrane of oral cavity, tongue, gum, or the like is observed by a test or the like; and an oral squamous-cell carcinoma tissue that has been confirmed to be derived from oral squamous-cell carcinoma and thus must be subjected to determination of malignancy or the stage progression of oral squamous-cell carcinoma.

In a case in which the deletion or inactivation of the PRTFDC 1 gene is observed in "a tissue obtained from a lesion in which suspected canceration of oral-cavity-derived tissues or cells is observed in a test or the like" by the detection method of the present invention, it is understood that such lesion tissue will reach (or has reached) the state of canceration so that the level of malignancy of the disease will increase. Thus, there is a demonstrated urgent need for implementation of a full-scale therapy (e.g., elimination of a lesion via a surgery or the like and full-scale chemotherapy). In addition, in a case in which the deletion or inactivation of the PRTFDC1 gene is observed in "an oral squamous-cell carcinoma tissue that has been confirmed to be derived from oral squamous-cell carcinoma and thus must be subjected to determination of malignancy or the stage progression of oral squamous-cell carcinoma," it is also understood that the level of malignancy of the cancerous tissue will increase. Thus, there is a demonstrated urgent need for implementation of full-scale therapy (e.g., elimination of a lesion via a surgery or the like and full-scale chemotherapy). An oral squamous-cell carcinoma tissue collected as a specimen may be subjected to necessary treatment such as preparation of DNA or RNA from the collected tissue followed by the detection method of the present invention.

### (2) Detection of deletion of PRTFDC 1 gene

Examples of a typical method by which PRTFDC1 gene deletion can be directly detected include a CGH (Comparative Genomic Hybridization) method and a FISH (Fluorescence in situ hybridization) method. According to the detection method in this embodiment, BAC (Bacterial Artificial Chromosome) DNA, YAC (Yeast Artificial Chromosome) DNA, or PAC (P1-drived Artificial Chromosome) DNA (hereinafter, also referred to as BAC DNA, for example) having the PRTFDC 1 gene is labeled and then FISH is performed, so that the presence or the absence of the PRTFDC 1 gene (that is, deletion of PRTFDC1 gene) can be detected. Specific examples of such BAC DNA having the PRTFDC1 gene include RP11-165A20 and the like.

It is preferable and practical to carry out the method in the above embodiment with the use of a genomic DNA-immobilized matrix.

The amount of BAC DNA or the like obtained in a conventional manner is so small that a large number of genomic DNA-immobilized matrices cannot be produced for practical application. Thus, it is necessary to obtain gene amplification products of such DNA. (A gene amplification process for this purpose is referred to as "infinite amplification" in some cases.) Upon infinite amplification, BAC DNA or the like is first digested with a four-base recognition enzyme such as *Rsa* I, *Dpn* I, *Hae* III, or the like, followed by ligation with the addition of an adaptor . An adaptor comprises oligonucleotides having 10 to 30 bases and preferably 15 to 25 bases. Double strands of such adaptor have sequences complementary to each other. After annealing, the 3' end of one of the oligonucleotides, at which a blunt end is formed, must be phosphorylated. Next, a primer having a sequence identical to the other oligonucleotide of the adaptor is used for amplification via PCR (polymerase chain reaction). Thus, infinite amplification can be carried out. Meanwhile, it is also possible to use, as a detection probe, an aminated oligonucleotide comprising 50 to 70 bases, which is inherent to BAC DNA or the like.

BAC DNAs or the like subjected to infinite amplification are immobilized on a matrix and preferably on a solid matrix. Accordingly, a desired DNA-immobilized matrix can be produced. An example of such solid matrix is more preferably a glass plate. Such a solid matrix made of glass or the like is more preferably coated via adhesion with poly-L-lysine, aminosilane, gold, aluminium, or the like.

The concentration of DNA subjected to infinite amplification to be spotted on a matrix is preferably 10 pg/µl to 5 µg/µl and more preferably 1 ng/µl to 200 ng/µl. The amount of the same to be spotted on the matrix is preferably 1 nl to 1 µl and more preferably 10 nl to 100 nl. In addition, the size and the shape of each spot that is immobilized on the matrix are not particularly limited. In terms of size, such spot may have a diameter ranging from 0.01 to 1 mm, for example. In addition, the shape of such spot may be a circle or ellipse from an overhead view. The thickness of a dry spot is not particularly limited; however, it may be 1 to 100 µm. Further, the number of spots is not particularly limited; however, it may be 10 to 50,000 spots and more preferably 100 to 5,000 spots on the matrix used. DNAs are spotted singly to quadruplicate. However, preferably, DNAs are spotted in duplicate or triplicate.

Regarding preparation of dry spots, it is possible to produce dry spots by, for example, spotting BAC DNAs or the like subjected to infinite amplification on a matrix with the use of a spotter, forming a plurality of spots thereon, and drying the spots. Examples of a spotter that can be used include an inkjet printer, a pin-array printer, and a bubble jet (trademark) printer. An inkjet printer is desirably used. For instance, GENESHOT (NGK INSULATORS; Nagoya, Japan) or the like can be used.

As described above, it is possible to produce a desired DNA-immobilized matrix by immobilizing BAC DNAs or the like subjected to infinite amplification onto a matrix, and preferably, onto a solid matrix.

In addition, an example of a means of directly detecting the deletion of the PRTFDC1 gene is the Southern blot method. The Southern blot method is a method for detecting the presence of the PRTFDC1 gene in a specimen by separating and immobilizing genomic DNA obtained from the specimen and detecting hybridization of such genomic DNA with the PRTFDC1 gene.

Furthermore, the deletion of the PRTFDC1 gene can also be detected by the Northern blot method or the real-time RT-PCR method. The Northern blot method comprises separating and immobilizing mRNA obtained from a specimen and detecting hybridization between the mRNA and the PRTFDC1 gene, so as to detect the presence of the mRNA of the gene in the specimen. The real-time RT-PCR method is a method for determining (in realtime) the amplification of a target gene as a result of a reverse transcription reaction and a polymerase chain reaction. By such method, mRNA serving as a template can be determined based on amplification rate. The determination is carried out using a fluorescent dye. There are two known methods: a method involving specific insertion (intercalation) of a dye (e.g., SYBR green) emitting fluorescence into double-stranded DNA; and a method involving a probe prepared by binding a fluorescent dye to an oligonucleotide specific to a DNA sequence to be amplified.

### (3) Detection of inactivation of PRTFDC1 gene

It has been reported that transcriptional inactivation occurs when a CpG-rich promoter and an exon region are densely methylated (Bird AP., et al., Cell, 99, 451-454, 1999). In the cases of cancer cells, CpG islands are frequently and densely methylated compared with other regions, and thus hypermethylation of a promoter region is deeply involved in the inactivation of an antioncogene of a cancer (Ehrlich M., et al, Oncogene, 21, 6694-6702, 2002).

As described below, CpG islands existing in an exon of the PRTFDC 1 gene was actually found to have promoter activities. In addition, the extent of methylation of the CpG islands strongly correlated with suppression of the expression of the PRTFDC1 gene in some oral squamous-cell carcinoma cases.

In addition, it was possible to demetylate such CpG islands by culturing such oral squamous-cell carcinoma cells in the presence of 5-azadeoxycytidine (5-aza-dCyd) serving as a demethylating reagent. As a result, it was possible to recover the expression level of the PRTFDC1 gene. Based on the above results, it has been revealed that highly frequent methylation (hypermethylation) of CpG islands is a cause of frequently occurring suppression of the expression of an antioncogene in squamous-cell carcinoma.

Recovery of the PRTFDC 1 gene expression level can be examined using the above detection means, specifically by causing a demethylating reagent (e.g., 5-azadeoxycytidine) to act on a cell specimen (a primary cancer cell derived from a cancerous tissue) that has been revealed to exhibit a decreased PRTFDC1 gene expression level. More specifically, when the expression level of the PRTFDC1 gene can be recovered by causing a demethylating reagent to act on a cell specimen, a factor that suppresses the gene in the cell specimen is methylation of CpG islands. Hence, a reasonable anti-tumor effect is expected with the administration of a drug having a demethylating effect to a specimen donor.

### (4) Method for inhibiting cell growth and cell growth inhibitor

According to the present invention, there are further provided a method for inhibiting cell growth which comprises introducing a PRTFDC1, c10orf63, THNSL1, GPR158, MYO3A, or GAF2 gene or a protein which is an expression product of such a gene into cells *in vitro,* and a cell growth inhibitor comprising said gene or protein.

For handling the PRTFDC1, c10orf63, THNSL1, GPR158, MY03A, or GAF2 gene, cDNAs obtained from cultured cells through publicly known methods to those skilled in the art may be used, or enzymatically synthesized ones through PCR method may be also used. When DNA is obtained through PCR method, PCR is performed using human chromosomal DNA or cDNA library as a template, and primers designed to amplify a nucleotide sequence of interest. DNA fragments amplified through PCR can be cloned in an appropriate vector which can proliferate in a host such as E. coli.

Manipulations such as preparation of detection probes or primers for the PRTFDC1, c10orf63, THNSL1, GPR158, MYO3A, or GAF2 gene and cloning of target genes are already known to those skilled in the art. For example, such manipulations can be performed according to methods described in Molecular Cloning: A Laboratory Mannual, 2nd Ed., Cold Spring Harbor Laboratory, Cold Spring Harbor, NY., 1989, Current Protocols in Molecular Biology, Supplement 1 to 38, John Wiley & Sons (1987-1997), or the like.

The PRTFDC1, c10orf63, THNSL1, GPR158, MYO3A, or GAF2 gene can be used in the form of a recombinant vector having such a gene incorporated therein. Examples of the vector to be used herein may include viral vectors and vectors for expression in animal cells. Preferably, viral vectors are used. Examples of such viral vector include retroviral vectors, adenoviral vectors, adeno-associated virus vectors, baculovirus vectors, vaccinia virus vectors, and lentivirus vectors. Of these, retroviral vectors are particularly preferred to use, since retroviral vectors enable stable and long-term expression of a foreign gene that had been incorporated into such vectors, through incorporation of the virus genome into a host chromosome after infection into cells.

Examples of the vector for expression in animal cells to be used herein may include pCXN2 (Gene, 108, 193-200, 1991), PAGE207 (JP Patent Publication (Kokai) No. 6-46841 (1994)) or variants thereof.

The above recombinant vector can be produced through transfection into an appropriate host to effect transformation, followed by culturing of thus obtained transformant. When the recombinant vector is a viral vector, animal cells capable of producing viruses are used as the host to be transfected with such a viral vector. For example, COS-7 cells, CHO cells, BALB/3T3 cells, and HeLa cells are use. Examples of the host to be used for retroviral vectors include ψCRE, ψCRIP, and MLV. Examples of the hosts to be used for adenoviral vectors or adeno-associated virus vectors include human embryonic kidney-derived 293 cells. Viral vectors can be transfected into animal cells by a calcium phosphate method. Moreover, when the recombinant vector is a vector for expression in animal cells, the E. coli K12 strain, the HB101 1 strain, and the DH5α strain, or the like can be used as the host to be transfected with such a vector. Transformation of E. coli is publicly known to those skilled in the art.

Thus obtained transformant is cultured in an appropriate medium under appropriate culture conditions, respectively. For example, a transformant of E. coli can be cultured using a liquid medium at a pH of about 5 to 8 containing carbon sources, nitrogen sources, inorganic substances, and the like which are essential for growth. The culture is normally carried out at 15°C to 43°C for about 8 to 24 hours. In this case, the recombinant vector of interest can be obtained through usual DNA isolation and purification methods, on completion of culture.

Moreover, transformants of animal cells can be cultured using a medium such as a 199 medium, an MEM medium, or a DMEM medium containing about 5% to 20% fetal bovine serum. The pH of the medium is preferably about 6 to 8. The culture is normally carried out at 30°C to 40°C for about 18 to 60 hours. In this case, since virus particles containing a target recombinant vector are released into a culture supernatant, the recombinant vector can be obtained through concentration and purification of the virus particles by a cesium chloride centrifugation method, a polyethylene glycol precipitation method, a concentration method using a filter, or the like.

The cell growth inhibitor of the present invention can be produced by mixing the abovementioned gene serving as an active ingredient with a base that is commonly used for gene therapeutic agents. Moreover, when such a gene is incorporated into a viral vector, virus particles containing the recombinant vector are prepared, and are then mixed with a base that is commonly used for gene therapeutic agents.

As to the base to be used for mixing the abovementioned gene or protein serving as an active ingredient, bases commonly used for injectable agents can be used. Examples thereof include: distilled water: salt solutions containing sodium chloride, a mixture of sodium chloride and mineral salts, or the like: solutions of mannitol, lactose, dextran, glucose, or the like: amino acid solutions of glycine, arginine, or the like: and mixed solutions having glucose solution with an organic acid or salt solution. Alternatively, these bases can also be prepared into injectable agents in the form of a solution, suspension, or dispersion, with use of auxiliary agents such as an osmoregulator, a pH adjuster, a vegetable oil, and a surfactant, in accordance with usual methods which are already known to those skilled in the art. These injectable agents can also be prepared in the form of a pharmaceutical preparation to be dissolved at the time of use, through operations such as powderization or lyophilization.

The form of administration of the cell growth inhibitor of the present invention may be either systemic administration such as usual intravenous administration and intraarterial administration, or local administration such as local injection and oral administration. Furthermore, administration of the cell growth inhibitor may also take a combined form with catheterization, gene introduction technology, or surgical operation.

The administration dose of the cell growth inhibitor of the present invention varies depending on the age and gender of the patient, the symptom, the administration route, the frequency of administration, and the dosage form. Generally, the daily dose for an adult is within a range of about 1 µg/kg of body weight to 1000 mg/kg of body weight, and preferably a range of about 10 µg/kg of body weight to 100 mg/ kg of body weight, in terms of weight of recombinant gene. The frequency of administration is not particularly limited.

### (5) Method for activating cell growth and cell growth activating agent

According to the present invention, there are provided a method for activating cell growth which comprises introducing an siRNA, an shRNA, an antisense oligonucleotide, or a loss-of-function type gene of the PRTFDC1, c10orf63, THNSL1, GPR158, MYO3A, or GAF2 gene into tumor cells *in vitro,* and a cell growth activating agent which comprises said siRNA, shRNA, antisense oligonucleotide, or loss-of-function type gene.

siRNA is a double-strand RNA having a length of about 20 nucleotides (for example, 21 to 23 nucleotides) or shorter. Expression of such an siRNA in a cell enables to suppress the expression of a gene targeted by the siRNA (PRTFDC1, c10orf63, THNSL1, GPR158, MYO3A, or GAF2 gene in the present invention).

The siRNA to be used in the present invention may take any form as long as it is capable of inducing RNAi. Here, the term "siRNA" is an abbreviation for "short interfering RNA", which refers to a short-chain double-strand RNA of 10 nucleotides or longer obtained by: chemical or biochemical synthesis in an artificial manner; *in vivo* synthesis; or *in vivo* degradation of double-strand RNA of about 40 nucleotides or longer. The siRNA normally has a structure comprising 5'-phosphoric acid and 3'-OH, where the 3' terminal projects by about 2 nucleotides. A specific protein binds to the siRNA to form RISC (RNA-induced-silencing-complex). This complex recognizes mRNA having the homologous sequence to that of siRNA and binds thereto. Then, the mRNA is cleaved at the central part of the siRNA with an RNase III-like enzymatic activity.

The siRNA sequence and the mRNA sequence being the target of cleavage preferably match 100%. However, such 100% match is not always required, when unmatched nucleotides are located away from the central part of the siRNA. This is because the RNAi cleaving activity often partially remains.

Preferably, the homologous region between the siRNA nucleotide sequence and the nucleotide sequence of the PRTFDC1, c10orf63, THNSL1, GPR158, MYO3A, or GAF2 gene whose expression has to be suppressed, does not include the translation initiation region of the concerned gene. Since various transcriptional factors and translational factors are predicted to bind to the translation initiation region, it is anticipated that the siRNA be unable to effectively bind to the mRNA, leading to lowered effect. Accordingly, the homologous sequence is preferably away from the translation initiation region of the concerned gene by 20 nucleotides, and more preferably by 70 nucleotides. The homologous sequence may be, for example, a sequence in the vicinity of the 3' terminal of the concerned gene.

According to another aspect of the present invention, an shRNA (short hairpin RNA) comprising a short hairpin structure having a projection at the 3' terminal may also be used as a factor which can suppress the expression of a target gene through RNAi. The term shRNA refers to a molecule of about 20 or more nucleotides, in which the single-strand RNA includes partially palindromic nucleotide sequences to thereby have a double-strand structure within the molecule, forming a hairpin-like structure. Such an shRNA is broken down into a length of about 20 nucleotides (typically 21 nucleotides, 22 nucleotides, and 23 nucleotides, for example) within a cell after being introduced into the cell, and thus is capable of inducing RNAi in a similar manner to that of siRNA. As described above, the shRNA induces RNAi in a similar manner to that of siRNA, and thus can be effectively used in the present invention.

The shRNA preferably has a projection at the 3' terminal. There is no particular limitation on the length of the double-strand portion, although it is preferably about 10 or more nucleotides, and more preferably about 20 or more nucleotides. Here, the projecting 3' terminal is preferably a DNA, more preferably a DNA of at least 2 or more nucleotides, and yet more preferably a DNA of 2 to 4 nucleotides.

As described above, in the present invention, siRNA or shRNA can be used as a factor which can suppress the expression of the PRTFDC1, c10orf63, THNSL1, GPR158, MYO3A, or GAF2 gene through RNAi. The advantages of siRNA are such that: (1) RNA itself, even when introduced into a cell, is not incorporated into a chromosome of normal cell, and therefore the treatment do not cause any inheritable mutations and the safety is high; (2) it is relatively easy to chemically synthesize short-chain double-strand RNA, and the form of double-strand RNA is more stable; and the like. The advantages of shRNA are such that: treatment through long-term suppression of gene expression can be achieved by producing a vector which can transcribe shRNA within a cell and introducing such a vector into the cell; and the like.

The siRNA or shRNA to be used in the present invention which can suppress the expression of the PRTFDC1, c10orf63, THNSL1, GPR158, MYO3A, or GAF2 gene through RNAi, may be chemically synthesized in an artificial manner, and may also be produced through *in vitro* RNA synthesis using DNA of a hairpin structure in which a sense strand DNA sequence and an antisense strand DNA sequence are linked in opposite directions, with a T7 RNA polymerase. In the case of *in vitro* synthesis, antisense and sense RNAs can be synthesized from a template DNA using the T7 RNA polymerase and a T7 promoter. After *in vitro* annealing thereof, transfection of the resultant RNA into cells induces RNAi to suppress the expression of a target gene. Here, for example, transfection of such RNA into cells can be carried out by a calcium phosphate method or a method using various transfection reagents (such as oligofectamine, lipofectamine, and lipofection).

The abovementioned siRNA and shRNA are also useful as cell growth activating agents. The administration method of the cell growth activating agent of the present invention may include oral administration, parenteral administration (such as intravenous administration, intramuscular administration, subcutaneous administration, intradermal administration, transmucosal administration, intrarectal administration, intravaginal administration, local administration to affected area, and skin administration), and direct administration to affected area. The agent of the present invention, if used as a medical composition, may be mixed with a pharmaceutically acceptable additive as required. Specific examples of such a pharmaceutically acceptable additive include, but not limited to, an antioxidant, a preservative, a coloring agent, a flavoring agent, a diluent, an emulsifier, a suspending agent, a solvent, a filler, an extending agent, a buffer agent, a delivery vehicle, a carrier, an excipient, and/or a pharmaceutical adjuvant.

The form of the pharmaceutical preparation of the agent of the present invention is not particularly limited, and examples thereof include a liquid agent, an injectable agent, and a sustained release agent. A solvent to be used for prescribing the agent of the present invention as the above pharmaceutical preparation may be either aqueous or non-aqueous.

Furthermore, the siRNA or shRNA serving as an active ingredient of the cell growth activating agent of the present invention can be administered in the form of a nonviral vector or a viral vector. In the case of a nonviral vector, there can be employed methods in which nucleic acid molecules are introduced using liposomes (such as a liposome method, an HVJ-liposome method, a cationic liposome method, a lipofection method, and a lipofectamine method), microinjection methods, methods in which nucleic acid molecules are transferred together with carriers (metal particles) into cells using a gene gun. If the siRNA or shRNA is administered *in vivo* using a viral vector, viral vectors such as a recombinant adenovirus and a recombinant retrovirus can be employed. Introduction of siRNA or shRNA gene into a cell or tissue can be achieved through introduction of DNA which expresses siRNA or shRNA into a detoxified DNA or RNA virus such as retrovirus, adenovirus, adeno-associated virus, herpes virus, vaccinia virus, poxvirus, poliovirus, Sindbis virus, Sendai virus, and SV40, followed by infection with the recombinant virus into the cell or tissue.

The dose of the cell growth activating agent of the present invention can be determined by those skilled in the art with a consideration of the purpose of administration, the disease severity, the age, weight, gender, and previous history of the patient, and the type of siRNA or shRNA serving as an active ingredient. The dose of siRNA or shRNA is not particularly limited, and examples thereof include about 0.1 ng/kg/day to about 100 mg/kg/day, and preferably about 1 ng/kg/day to about 10 mg/kg/day. RNAi effect is typically exerted for one to three days after the administration. Therefore, administration is preferably performed at a frequency of everyday to every third day. When an expression vector is used, the administration can be performed approximately once a week.

In the present invention, an antisense oligonucleotide can also be used as a cell growth activating agent. Antisense oligonucleotides to be used in the present invention are nucleotides that are complementary or hybridize to consecutive 5 to 100 nucleotide sequences within the DNA sequence of the PRTFDC1, c10orf63, THNSL1, GPR158, MY03A, or GAF2 gene. Such an antisense oligonucleotide may be either DNA or RNA, or may also be modified as long as its functions remain unaffected. The term "antisense oligonucleotide" used in this description includes not only oligonucleotides wherein all nucleotides corresponding to nucleotides composing a predetermined DNA or mRNA region are complementary to their counterparts, but also oligonucleotides that contain some mismatching nucleotides, as long as such oligonucleotides can stably hybridize to DNA or mRNA.

In addition, the antisense oligonucleotides may be modified. After appropriate modification, resulting modified antisense oligonucleotides will be hardly degraded *in vivo.* This enables more stable inhibition of ITIIα. Examples of such modified oligonucleotide include S-oligo type (phosphorothioate-type), C-5 thyazole type, D-oligo type (phosphodiester-type), M-oligo type (methylphosphonate-type), peptide nucleic acid type, phosphodiester binding type, C-5 propinyl pyrimidine type, 2-O-propylribose, and 2'-methoxyribose type antisense oligonucleotides. Furthermore, such antisense oligonucleotide may also be an antisense oligonucleotide wherein at least some of the oxygen atoms composing phosphate groups are substituted with sulfur atoms or otherwise modified. Such an antisense oligonucleotide is particularly excellent in terms of nuclease resistance, water solubility, and affinity for RNA. As such an antisense oligonucleotide wherein at least some of the oxygen atoms composing phosphate groups are substituted with sulfur atoms or otherwise modified, an S-oligo type oligonucleotide can be enumerated.

The number of nucleotides in such antisense oligonucleotide is preferably 50 or less and more preferably 25 or less. Too large number of nucleotides results in increased effort and cost in oligonucleotide synthesis and lowered yields. Furthermore, the number of nucleotides of such antisense oligonucleotide is 5 or more and preferably 9 or more. A number of nucleotides of 4 or less is undesirable because of the resulting lowered specificity to a target gene.

Such antisense oligonucleotide (or a derivative thereof) can be synthesized by a usual method. For example, an antisense oligonucleotide or a derivative thereof can be easily synthesized using a commercially available DNA synthesizer (such as one produced by Applied Biosystems). It can be obtained by a synthesis method such as a solid-phase synthesis method using phosphoroamidite or a solid-phase synthesis method using hydrogen phosphonate.

When an antisense oligonucleotide is used as a cell growth activating agent in the present invention, it is generally provided in the form of a medical composition containing the antisense oligonucleotide and additive(s) for pharmaceutical preparation (such as a carrier and an excipient). The antisense oligonucleotide can be administered as a medicament to mammals including humans. The route of administration for such an antisense oligonucleotide is not particularly limited and may be either of oral administration or parenteral administration (such as intramuscular administration, intravenous administration, subcutaneous administration, peritoneal administration, transmucosal administration in the nasal cavity or the like, and inhalation administration).

The form of the pharmaceutical preparation of such an antisense oligonucleotide is not particularly limited. Examples of the pharmaceutical preparation for oral administration include tablets, capsules, fine granules, powders, granules, liquids, and syrups. Examples of the pharmaceutical preparation for parenteral administration include injections, infusions, suppositories, inhalants, transmucosal absorption systems, transdermal absorption systems, nasal drops, and ear drops. The form of a drug containing the antisense oligonucleotide, additive(s) to be used for the pharmaceutical preparation, a method for producing the pharmaceutical preparation, and the like can be appropriately selected by those skilled in the art.

The dose of the antisense oligonucleotide can be appropriately determined with a comprehensive consideration of the gender, age, and weight of the patient, the symptom severity, the purpose of administration such as prevention or treatment, and the presence/absence of other complication symptoms. The dose is generally 0.1 µg/kg of body weight/day to about 100 mg/kg of body weight/day, and preferably 0.1 µg/kg of body weight/day to about 10 mg/kg of body weight/day.

Furthermore, in the present invention, a loss-of-function type gene of the PRTFDC1, c10orf63, THNSL1, GPR158, MY03A, or GAF2 gene can also be used as a cell growth activating agent. The loss-of-function type gene refers to a mutated gene which causes loss of function of the corresponding gene. Specific examples thereof include genes which translate proteins lacking their original functions, generally called muteins, including those lacking at least one constituent amino acid(s), those having at least one constituent amino acid(s) replaced by other amino acid(s), and those added with at least one amino acid(s), within the amino acid sequence produced by the concerned gene.

When such a loss-of-function type gene is used as the cell growth activating agent, it can be produced by mixing the abovementioned gene serving as an active ingredient with a base that is commonly used for gene therapeutic agents. Moreover, when such a gene is incorporated into a viral vector, virus particles containing the recombinant vector are prepared, and are then mixed with a base that is commonly used for gene therapeutic agents.

As to the base, bases commonly used for injectable agents can be used. Examples thereof include: distilled water: salt solutions containing sodium chloride, a mixture of sodium chloride and mineral salts, or the like: solutions of mannitol, lactose, dextran, glucose, or the like: amino acid solutions of glycine, arginine, or the like: and mixed solutions having glucose solution with an organic acid solution or salt solution. Alternatively, these bases can also be prepared into injectable agents in the form of a solution, suspension, or dispersion, with use of auxiliary agents such as an osmoregulator, a pH adjuster, a vegetable oil, and a surfactant, in accordance with usual methods which are already known to those skilled in the art. These injectable agents can also be prepared in the form of a pharmaceutical preparation to be dissolved at the time of use, through operations such as powderization or lyophilization.

The form of administration of the loss-of-function allele may be either systemic administration such as usual intravenous administration and intraarterial administration, or local administration such as local injection and oral administration. Furthermore, administration may also take a combined form with catheterization, gene introduction technology, or surgical operation.

The administration dose of the loss-of-function type gene varies depending on the age and gender of the patient, the symptom, the administration route, the frequency of administration, and the dosage form. Generally, the daily dose for an adult is within a range of about 1 µg/kg of body weight to 1000 mg/kg of body weight, and preferably a range of about 10 µg/kg of body weight to 100 mg/kg of body weight, in terms of weight of recombinant gene. The frequency of administration is not particularly limited.

Moreover, the abovementioned various gene therapeutic agents of the present invention can also be produced by adding a gene into a suspension of liposomes prepared by a usual method, followed by freezing and subsequent thawing. Examples of the method for preparing liposomes include a membrane shaking method, a sonication method, a reverse phase evaporation method, and a surfactant removal method. The suspension of liposomes is preferably subjected to sonication treatment before addition of a gene, so as to improve the efficiency of encapsulation of the gene. The liposomes having the gene encapsulated therein may be intravenously administered either directly or in the form of a suspension with water, physiological salt solution, or the like.

### (6) Screening method of antitumor substance

As described above, it is considered that inactivation of the PRTFDC1, c10orf63, THNSL1, GPR158, MY03A, or GAF2 gene serves as a main causative factor of oral squamous-cell carcinoma, and that drugs which normalize functions of such genes can be used as antitumor agents for oral squamous-cell carcinoma. In particular, if the causative factor of such inactivation is methylation of the CpG island of the PRTFDC1 gene, drugs capable of relieving/relaxing these causative factors are useful as antitumor agents.

As the premise for performing these present screening methods, oral squamous-cell carcinoma cells showing lowered expression level of the PRTFDC1 gene in specimen cells have to be obtained. In other words, the present screening method requires an "oral squamous-cell carcinoma cell line showing lowered expression of the PRTFDC1 gene due to methylation of CpG island of the PRTFDC1 gene". The method for establishing these cell lines can be carried out in accordance with usual methods, based on the abovementioned understandings. For example, such desirable "oral squamous-cell carcinoma cell line showing lowered expression of the PRTFDC 1 gene due to methylation of CpG island of the PRTFDC1 gene (hereinunder, also referred to as methylated cancer cell line)" can be established by the following manner. Among at least cells exhibiting inactivation of the PRTFDC1 gene, cells showing restoration of the PRTFDC1 gene level by treatment with an already-known demethylating reagent (such as 5-azadeoxycytidine) are selected, and subjected to passage culture.

In the present screening method, test substances have to be contacted with the abovementioned methylated cancer cell line. The form of such contact is not specifically limited. The contact can be achieved by adding a test substance, preferably diluted at an appropriate dilution strength, to the culture product of the methylated cell line, and subsequent culturing thereof. The PRTFDC1 gene expression level in the methylated cancer cell line is quantified before and after addition of the test substance. Preferably, the quantification is performed with the passage of time. The difference in the gene expression level throughout the quantification is compared to that of a control culture product that has been cultured without addition of the test substance under the same condition. If the gene expression level of the culture product with addition of the test substance is higher than that of the control culture product, the test substance is selected as an "antitumor substance capable of activating the PRTFDC1 gene through demethylation of CpG island of the PRTFDC1" in the reagent using a methylated cancer cell line.

Furthermore, preferably, the number of substances to be screened for as desirable antitumor ingredients for oral squamous-cell carcinoma by the present screening method is narrowed down to final candidates through additional *in vivo* screening, such as a screening method in which a growth inhibitory effect on oral squamous-cell carcinoma cells in nude mice transplanted with the abovementioned methylated cancer cell line, and improvement in the viability of such nude mice, are used as indexes.

The present invention is hereafter described in greater detail with reference to the following examples, although the technical scope of the present invention is not limited thereto.

### Examples

### Example 1: Gene alteration in oral squamous-cell carcinoma

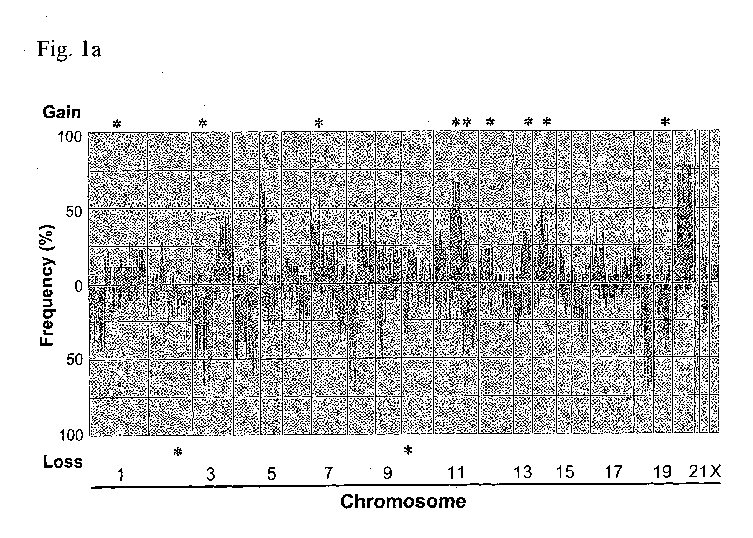

To detect a novel gene alteration in oral squamous-cell carcinoma, CGH array analysis was made using genomic DNAs prepared from 18 types of oral squamous-cell carcinoma cell line (OM-1, OM-2, TSU, ZA, NA, Ca9-22, HOC-313, HOC-815, HSC-2, HSC-3, HSC-4, HSC-5, HSC-6, HSC-7, KON, SKN-3, HO-1-N-1, and KOSC-2), and using MGC Cancer Array-800, and MGC Whole Genome Array-4500 (Fig. 1a). A genome extracted from the cell line (RT7) derived from normal oral epithelium was labeled with Cy5 as a control. Genomic DNAs prepared from the above oral squamous-cell carcinoma cell lines were used as test DNAs, and were labeled with Cy3. Specifically, each genomic DNA (0.5 µg) digested with *Dpn* II was labeled using a BioPrime Array CGH Genomic Labeling System (Invitrogen) in the presence of 0.6 mM dATP, 0.6 mM dTTP, 0.6 mM dGTP, 0.3 mM dCTP, and 0.3 mM Cy3-dCTP (oral squamous-cell carcinoma cells) or 0.3 mM Cy5-dCTP (normal cells). Cy3- and Cy5-labeled dCTPs were obtained from GE Healthcare. Ethanol was added so that the genomic DNA labeled with Cy3 or Cy5 was precipitated in the presence of Cot-1 DNA (Invitrogen). The resultant was dissolved in 120 µl of a hybridization mixture (50% formamide, 10% Dextran sulfate, 2xSSC (1xSSC: 150 mM NaCl/15 mM Sodium Citrate), 4% sodium dodecyl sulfate, pH 7.0). After 30 minutes of incubation at 37°C, the resultant was introduced onto a CGH array set in a hybridization machine (GeneTAC; Harvard Bioscience), followed by 48 to 72 hours of hybridization. Subsequently, the CGH array was washed in a 50% formamide/2xSSC (pH 7.0) solution at 50°C for 15 minutes and then washed at 50°C for 15 minutes in 2xSSC/0.1% SDS. After air-drying, the CGH array was monitored for fluorescence derived from Cy3 and Cy5 using GenePix 4000B scanner (Axon Instruments, CA, U.S.A.). The thus obtained results were analyzed using a GenePix Pro 6.0 imaging software (Axon Instruments, CA, U.S.A.). The average fluorescence intensity derived from Cy3 was adjusted to be the same as that of fluorescence intensity derived from Cy5, thereby determining the ratio of Cy3 to Cy5. When a genome has no abnormality, the resulting ratio is 1 (log2 ratio = 0). Determination was performed as follows. A ratio of 1.32 (or higher) (log2 ratio = 0.4 or more) indicates the presence of genome amplification, and a ratio of 4 (or higher) (log2 ratio = 2.0 or more) indicates the confirmation of significant amplification. A ratio of 0.75 (or lower) (log2 ratio = -0.4 or less) indicates possible heterozygote deletion in the genome and a ratio of 0.25 (or lower) (log2 ratio = -2 or less) indicates an extremely high possibility of homozygote deletion in the genome. Table 1 and Table 2 show the results.

**Table 1. High-level amplifications (log2ratio > 2.0) detected in OSCC cell lines by CGH-array analysis using MCG Cancer Array-800 and Whole Genome Array-4500.**

| No. | BAC | Locus^{a} | | Cell line (Total 18) | | Possible candidate gene^{b} | Number of Known gene |
|---|---|---|---|---|---|---|---|
| | | Chr. Band | Position | n | Name | | |
| 1 | RP11-123P3 | 1q21 | chr1:144,160,737-144,316,863 | 1 | HSC-7 | *BCL9* | 38 |
| | RP11-433J 22 | | chr1:144,332,334-144,519,348 | | HSC-7 | | |
| | | | | | | | |
| 2 | RP11-444P10 | 3p13 | chr3:69,701,828-69,903,040 | 1 | HSC-7 | *MITF* | 20 |
| | RP11-215K24 | | chr3:70,082,272-70,235,455 | | | | |
| | RP 11-90H 15 | | chr3:71,172,843-71,304,638 | | | | |
| | RP11-154H23 | | chr3:71,622,959-71,816,508 | | | | |
| | | | | | | | |
| 3 | RP11-14K11 | 7p11.2 | chr7:54,903,388-54,903,650 | 4 | ZA NA HSC-2 KON | *EGFR* | 9 |
| | RP11-339F13 | | chr7:55,029,594-55,154,846 | | | | |
| | RP11-97P11 | | chr7:55,238,305-55,381,999 | | | | |
| | RP11-34J 24 | | chr7:55,274,090-55.403,627 | | | | |
| | | | | | | | |
| 4 | RP11-120E20 | 11p15.4 | chr11:3,573,460-3,758,006 | 1 | OM-1 | | 62 |
| | | | | | | | |
| 5 | RP11-350M7 | 11p15.2 | chr11:13,284,758-13,494,094 | 1 | HSC-6 | *PTH* | 8 |
| | | | | | | | |
| 6 | RP11-30016 | 11q13.3 | chr11:69,162,462-69,323,966 | 1 | OM-1 | *BCL1 , FGF4, CCNDI, FGF3* | 40 |
| | CTD-2234J21 | | chr11:69,307,612-69,307,884 | | | | |
| | RP11-804L21 | | | | | | |
| | | | | | | | |
| 7 | RP11-681017 | 11q22 | chr11:100,365,386-100,536,934 | 1 | TSU | *PGR, YAPI , CIAP1 , MMP7, MMP1* | 31 |
| | RP11-111A13 | | chr11:101,517,505-101,686,954 | | | | |
| | RP11-864G5 | | chr11:101,600,598-101,786,581 | | | | |
| | RP11-31506 | | chr11:101,724,636-101,939.137 | | | | |
| | RP11-750P5 | | chr11:102,010,611-102,191,046 | | | | |
| | RP11-2122 | | chr11:102,613,696-102,771,594 | | | | |
| | | | | | | | |
| 8 | RP11-117K21 | 11q23.3 | chr11:119,773,356-119,939,639 | 2 | OM-1 NA | | 3 |
| 9 | CTD-2058B01 | 12p13 | | 1 | HSC-7 | *CCND2, FGF6* | 33 |
| | RP11-24N12 | | chr12:2,977,826-3,145,569 | | | | |
| | RP11-88D16 | | chr12:3,295,578-3,473,577 | | | | |
| | RP11-74M9 | | chr12:4,177,493-4,279,837 | | | | |
| | RP11-388F6 | | chr12:4,313,654-4,483,853 | | | | |
| | | | | | | | |
| 10 | RP11-87C12 | 12q24.31 | chr12:120,771,781-120,958,360 | 1 | HSC-5 | *BCL7A* | 56 |
| | RP11-512M8 | | chr12:121,153,004-121,339.958 | | | | |
| | | | | | | | |
| 11 | RP11-313L9 | 13q33.3-q34 | chr13:109,059,437-109,253,923 | 1 | HSC-7 | *DPT, GAS6* | |
| | RP11-91C11 | | chr13:110,332,616-110,333,140 | | | | |
| | RP11-230F18 | | chr13:113,193,310-113,252,507 | | | | |
| | RP11-51H10 | | chr13:113,492,714-113,658,045 | | | | |
| | | | | | | | |
| 12 | RP11-74M3 | 14q24.1 | chr14:68,410,041-68,581,432 | 1 | NA | | 25 |
| | RP11-59M15 | | chr14:69,101,595-69,152,329 | | | | |
| | RP11-72J8 | | chr14:69,266,041-69,440,807 | | | | |
| | RP11-18G18 | | chr14:69,652,605-69,652,944 | | | | |
| | | | | | | | |
| 13 | RP11-91H20 | 19q13 | chr19:43,015,766-43,182,195 | 1 | SKN-3 | *SUPT5H* | 99 |
| | RP11-140E1 | | chr19:43,372,949-43,537,375 | | | | |
| | RP11-118P21 | | chr19:43,861,826-43,880,848 | | | | |
| | RP11-446K10 | | chr19:44,156,778-44,353,574 | | | | |
| | RP11-67A5 | | chr19:44,449,022-44,620,150 | | | | |
| | RP11-25609 | | chr19:44,586,344-44,776,946 | | | | |
| | | | | | | | |
| 14 | RP11-89A2 | 22q12.1 | chr22:24,445,961-24,605,516 | 1 | HSC-7 | | 13 |
| | RP11-291 20 | | chr22:24,942,081-24,951,525 | | | | |

| | | | | | | | |
|---|---|---|---|---|---|---|---|
| "Based on UCSC Genome Browser, May 2004 Assembly ^{b}Representative candidate oncogene located around BAC | | | | | | | |

**Table 2. Homozygous deletions (log2ratio < -2.0) detected in OSCC cell lines by CGH-array analysis using MCG Cancer Array-800 and Whole Genome Array-4500**

| No. | BAC | Locus^{a} | | Cell line (Total 18) | | Possible candicate gene ^{b} | Number of Known gene |
|---|---|---|---|---|---|---|---|
| | | Chr. Band | Position | n | Name | | |
| 1 | RP11 91K6 | 2q24.1-q24.2 | chr2:159,678,005-159,832,799 | 1 | HO-1-N1 | none | 23 |
| | | | | | | | |
| 2 | RP11 165A20 | 10p12.1 | chr10:25,269,606-25,426,074 | 1 | HSC-6 | none | 17 |
| | RP11 80K21 | | chr10:25,407,097-25,570,777 | | | | |
| | RP11 66P13 | | chr 10:26,528,582-26,697,629 | | | | |

| | | | | | | | |
|---|---|---|---|---|---|---|---|
| ^{a}Based on UCSC Genome Browser, May 2004 Assembly ^{b}Genes located around BAC, whose homozygous deletion was validated by genomic PCR. | | | | | | | |

High-level gene amplification could be confirmed for 10 out of 18 types of oral squamous-cell carcinoma (55.6%) and in 15 gene loci. Moreover, gene deletion could be confirmed for 2 out of 18 types of oral squamous-cell carcinoma and in 2 gene loci.

### Example 2: Isolation of gene contained in deletion region of chromosome 10p12 of oral squamous-cell carcinoma

To determine a gene contained in a homozygous deletion region of chromosome 10p12 in oral squamous-cell carcinoma cells (HSC-6), the range of the homozygous deletion region was first determined by genomic PCR using HSC-6 cells. Table 3 shows primer sequences used in genomic PCR.

**TABLE 3**

| Supplementary Table 2. Primer sequences used in this study | | | |
|---|---|---|---|
| Method | Target | Forward primer | Reverse primer |
| Genomic PCR | | | |
| | *ARHGAP21* | 5'-CACCTTGGCTTCAACAAACAG | 5'-GGTTGGGCATTCGCTGCAG |
| | *PRTFDC1* | 5'-CTGGCCAAGGATATTATGAAAG | 5'-CCTTCATTGAGACAAATCGATC |
| | *c10orf63* | 5'-TATACCAAAGAAGATCCGCAAG | 5'-GTGTTCTAATAAATATAACGGTTAT |
| | *THNSL1* | 5'-ATGCATTACCTCCACTGCATG | 5'-GATTTTGGACAAGTTGTTCCAC |
| | *GPR158* | 5'-ATATTGCTACAGAAGCATATGAG | 5'-TCCTCTGGATCCAAGCTGTG |
| | *MYO3A* | 5'-TTTGCCCAGTCGTTCTGGAC | 5'-TTTACCTTTCATTAGCCTTAATAC |
| | *GAD2* | 5'-TCCAGTGATTAAAGCCAGAATG | 5'-TTGCCGCTGGGTTTGAGATG |
| | *APBB1IP* | 5'-CAAGAGGCAAGAGAACCCAG | 5'-AGGACACGTTGCCTCTCTTC |
| | *GAPDH* | 5'-AACGTGTCAGTGGTGGACCTG | 5'-AGTGGGTGTCGCTGTTGAAG |

| RT-PCR | | | |
|---|---|---|---|
| | *PTFDC1* | 5'-GTTGGTGAAGAGAACATCCAG | 5'-TCAGATCTCTGAAGTATTCATTG |
| | *c10orf63 THNSL1* | 5'-TATACCAAAGAAGATCCGCAAG 5'-ATTACCTCCCTTGACGGCTC | 5'-AAAGTTGTGCTGTTGGTATCATG 5'-GAAATCGCTGTGCATGTTTATC |
| | *GPR158* | 5'-ATATTGCTACAGAAGCATATGAG | 5'-ATATTTCCAGTTGGGCATAGAG |
| | *MYO3A* | 5'-GAGAAGCATGTGGTTTGGCA | 5'-ATTGGCTTATGGTGCGAGAC |
| | *GAD2* | 5'-AGAGAGAATGAGTCGCCTCT | 5'-TTGCCGCTGGGTTTGAGATG |
| | *GAPDH* | 5'-AATTAGATTTTGGTGGTATTGG | 5'-ACCCTTTCCCCTCTCCTAC |

| COBRA and Bisulfite Sequencing | | | |
|---|---|---|---|
| | region1 | 5'-TGGATTTGGGAATTAGGGGTT | 5'-ACCCCAAAATAAAAAATTTACATTT |
| | region2 | 5'-AAATGTAAATTTTTTATTTTGGGGT | 5'-CCCAAAAAAAAAAAAACCTCCAA |

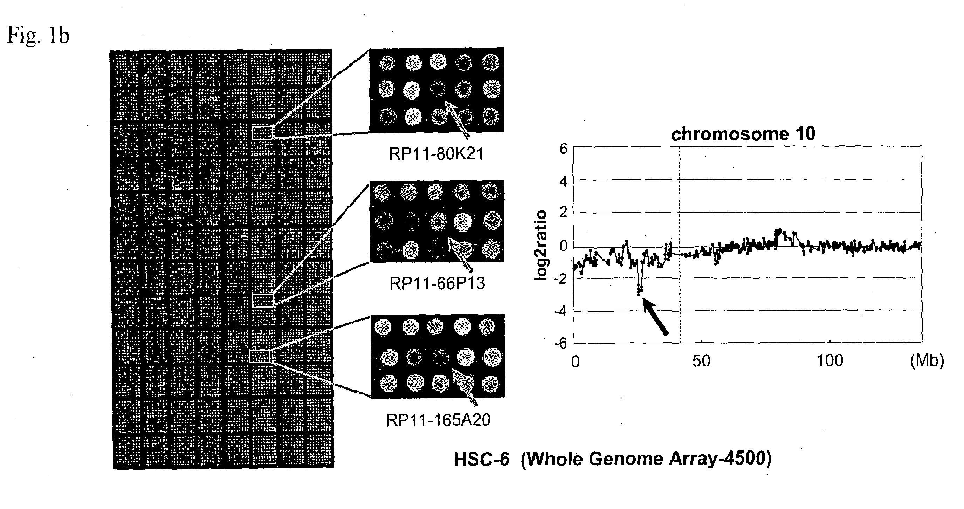

The deletion region could be confirmed to be an approximately 2.95-Mb deletion region at maximum based on the results of CGH array and the human genome database (http://genome. ucsc. edu/) (Fig. 1b and Fig. 2a). Subsequently, the presence of 7 genes was confirmed in this region.

Of these genes, homozygous deletion of the PRTFDC1 gene, the c10orf6 gene, the THNSL1 gene, the GPR158 gene, the MY03A gene, and the GAD2 gene could be confirmed only in HSC-6 cells (1/18, 5.6%; Fig. 2b). However, the ARHGAP21 gene was found to have remained undeleted.

### Example 3: Disappearance of PRTFDC1 mRNA expression in oral squamous-cell carcinoma cell lines

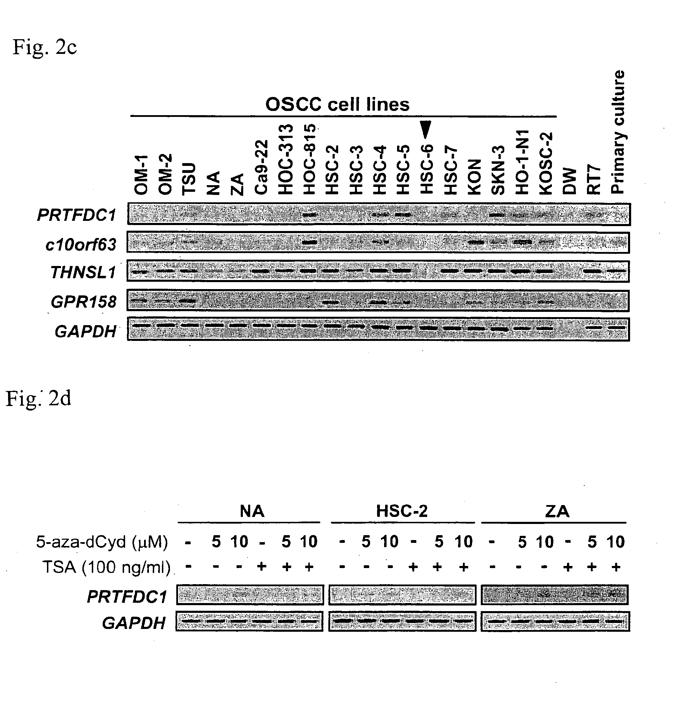

To examine the expression levels of the 6 above genes (the PRTFDC1 gene, the c10orf6 gene, the THNSL1 gene, the GPR158 gene, the MYO3A gene, and the GAD2 gene), 18 types of oral squamous-cell carcinoma cell line and normal oral epithelium cells were subjected to reverse transcriptase (RT)-PCR. Specifically, a single-chain cDNA was synthesized from the RNA extracted from each cultured cell line using the SuperScript First-Strand Synthesis System (Invitrogen). PCR was then performed using primer sequences listed in Table 4 (supplementary Table 1). Moreover, a glyceraldehyde-3-phosphate dehydrogenase (GAPDH) gene was used as a control. As a result, complete disappearance of PRTFDC 1 mRNA expression could be confirmed in 8 cell lines in which homozygous deletion of chromosomal region 10p12 should not have taken place, as in the case of HSC-6 cells (Fig. 2c). Furthermore, HOC-313 cells showed a more significant decrease (1/18, 5.6%) in expression compared with RT7 cells, the primary cells obtained from the normal mucous membrane of the oral cavity. Such disappearance of PRTFDC1 mRNA expression in the oral squamous-cell carcinoma cell lines may be due to a mechanism other than genomic deletion, such as an epigenetic phenomenon. On the other hand, disappearance of the expression of or decreases in the expression levels of c10orf63, THNSL1, and GPR158 genes were found to take place at low frequencies.

MYO3A gene and GAD2 gene expression could not be confirmed in either RT7 cells or primary cells obtained from the normal mucous membrane of the oral cavity. Based on the results of examination using the expression database (ncbi. nlm. nih. gov and http://www.lsbm.org/database/index.html), this may be because these genes exhibiting tissue-specific expression patterns were not expressed in oral-cavity-derived cells.

### Example 4: Effects of demethylation on PRTFDC 1 gene expression

To examine if the suppressed expression of the PRTFDC1 gene was due to DNA methylation, oral squamous-cell carcinoma cell lines not expressing the PRTFDC1 gene were treated using a 1 µM or 5 µM demethylating reagent 5 - aza - dCyd for 5 days and/or using deacetylation inhibitory agent TSA (100 ng/ml) for 24 hours. RNAs were extracted from these cells and then PRTFDC1 gene expression was examined by RT-PCR (Fig. 2d). As a result, it was revealed that the PRTFDC 1 gene recovers its gene expression by treatment with 5-aza-dCyd. It was clearly assumed based on this result that DNA methylation is involved in suppression of the expression of the PRTFDC1 gene. Moreover, treatment with TSA resulted in no differences in PRTFDC1 gene expression. Hence, it was revealed that histone deacetylation lightly affects the regulation of PRTFDC1 gene expression.

### Example 5: Promoter activity of the PRTFDC CpG island

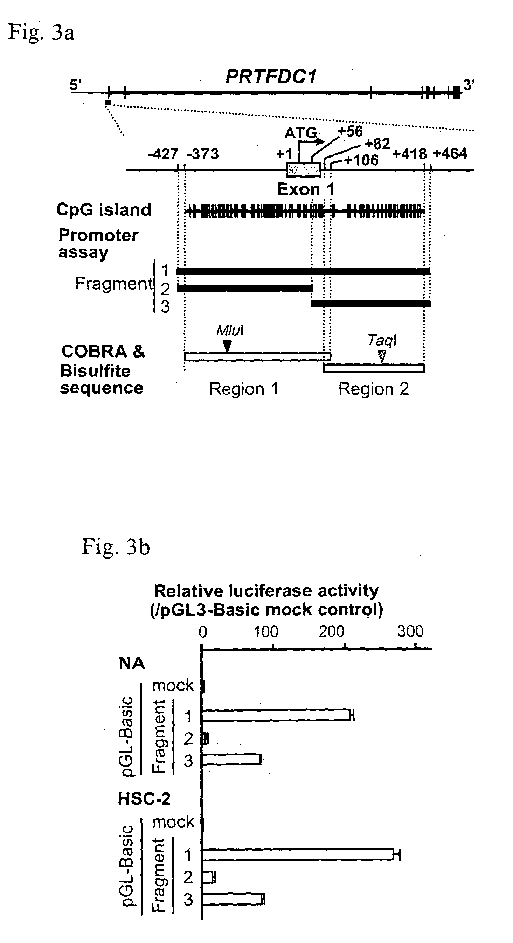

Methylation of a CpG island is one mechanism that suppresses gene expression. The CpG island of the PRTFDC1 gene was analyzed using the CpGPLOT program (http://www. ebi. ac. uk/emboss/cpgplot/). As a result, the presence of the CpG island in the periphery of exon 1 of the PRTFDC1 gene was confirmed (Fig. 3a). To examine the promoter activity of the CpG island, the CpG island was divided into 3 fragments (Fragment 1, Fragment 2, and Fragment 3) including the peripheral portion thereof. These fragments were inserted into luciferase reporter plasmids (pGL3 - Basic vector: Promega). Oral squamous-cell carcinoma cell lines (NA and HSC-2) were then transfected with the plasmids (Fig. 3a). Luciferase activity was measured according to the manuals using a Dual-Luciferasereporter assay system (Promega). Thus, luciferase activity derived from each pGL3 vector having each fragment was measured (Fig. 3b). As a result, Fragments 1 and 3 were found to have high luciferase activities (Fig. 3b).

### Example 6: Methylation status of the PRTFDC1 CpG island in oral squamous-cell carcinoma cell lines and oral squamous-cell carcinoma clinical specimens

To actually confirm the methylation status of the CpG island of exon 1 of the PRTFDC 1 gene in oral squamous-cell carcinoma cell lines, the above 2 regions (Fragment 1 and Fragment 2) were analyzed using a combined bisulfite restriction analysis (COBRA) method (Fig. 3a).

Specifically, genomic DNA (2 µg) derived from each oral squamous-cell carcinoma cell line was treated in sodium bisulfite at 50°C overnight using an EZ DNA methylation kit (Zymo RESEARCH, CA, U.S.A.). PCR was performed using primers (supplementary Table 1) designed so as to amplify target regions. The thus obtained PCR products were digested with a *Mlu* I restriction enzyme (New England BioLabs) or a *Taq* I restriction enzyme (New England BioLabs). *Mlu* I or *Taq* I has a feature of not digesting a unmethylated sequence modified with sodium bisulfite, but digesting a methylated sequence not modified with sodium bisulfite. With the use of such feature, the degrees of methylation were monitored. After electrophoresis of PCR fragments, the ratio of the concentration of a band of a methylated fragment to that of a band of an unmethylated fragment was measured by densitometry using MultiGauge 2.0 (FUJIFILM Corporation). The degree of methylation of a methylated region was represented in percentage terms. Furthermore, these sequences were subcloned into TOPO TA cloning vectors (Invitrogen) so that the nucleotide sequences were determined.

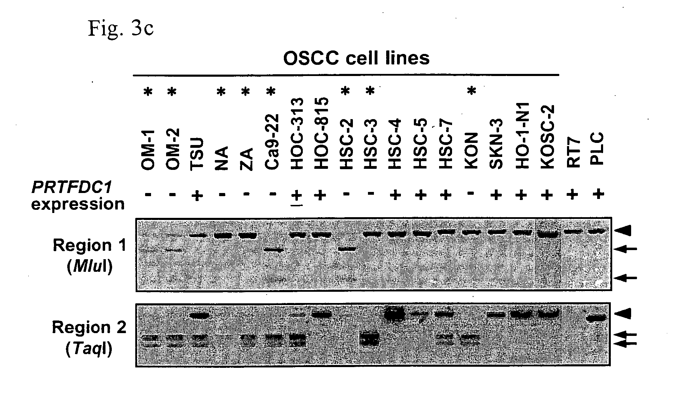

As a result, cell lines (TSU, HOC-815, HSC-4, HSC-5, HSC-7, SKN-3, HO-1-N1, and KOSC-2) expressing the PRTFDC1 gene and RT7 cells were found to tend to show low degrees of methylation dominantly in both regions (Fig. 3c). However, two regions were confirmed to be both dominantly and frequently methylated in cell lines not expressing the PRTFDC1 gene or expressing the same at low levels (Fig. 3c). In particular, Region 2 was methylated frequently. Based on the results of the above promoter assay, it is considered that methylation of Region 2 plays an important role in suppression of PRTFDC1 gene expression.

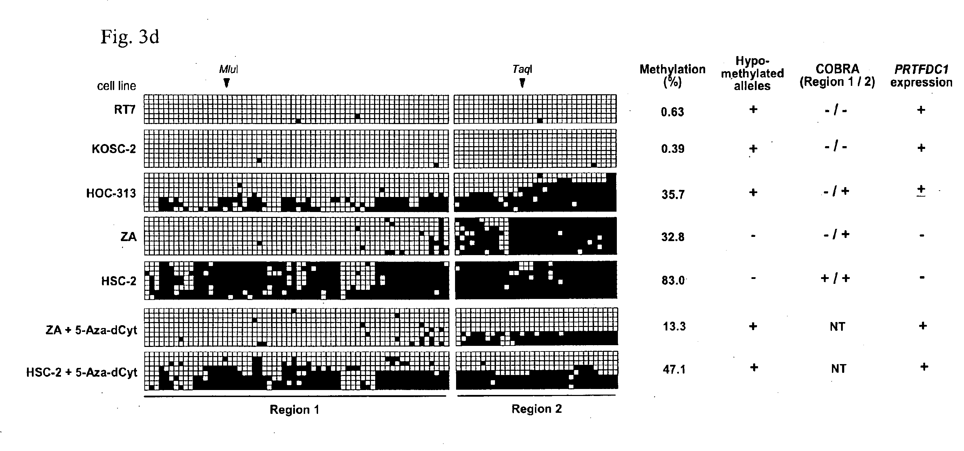

Further analysis was undertaken by confirming the degree of methylation of the CpG island of the PRTFDC1 gene by a bisulfite sequencing method (Toyota M., et al., Cancer Res. 59, 2307, 1999) (Fig. 3d). As a result, the CpG site of the PRTFDC1 gene was found to exhibit an extremely high tendency of methylation in cell lines (Table 4) such as ZA and HSC-2 in which homozygous deletion of the PRTFDC1 gene had not taken place but in which the PRTFDC1 gene expression had disappeared. Furthermore, the CpG site was found to be unmethylated in the cell lines expressing the PRTFDC 1 gene, such as RT7 and KOSC-2.

**Table 4: Supplementary Table 1.**

| Status of genomic copy-number, mRNA expression, and CpG island hypermethylation of the *PRTFDC1* gene | | | |
|---|---|---|---|
| Cell line | *PRTFDC1* | | |
| | Deletion | mRNA expression | CpG island hypermethylation^{a} |
| OM-1 | - | - | + |
| OM-2 | Hemizygous | - | + |
| TSU | - | + | + |
| NA | Hemizygous | - | + |
| ZA | Hemizygous | - | + |
| Ca9-22 | - | - | + |
| HOC-313 | - | ± | + |
| HOC-815 | - | + | + |
| HSC-2 | - | - | + |
| HSC-3 | - | - | + |
| HSC-4 | - | + | - |
| HSC-5 | - | + | - |
| HSC-6 | Homozygous | | NT^{b} |
| HSC-7 | - | + | + |
| KON | - | - | + |
| SKN-3 | Hemizygous | + | - |
| HO-1-N1 | - | + | - |
| KOSC-2 | - | + | - |

| | | | |
|---|---|---|---|
| ^{a}Methylation status was determined by COBRA as described in Materials and Methods. ^{b}NT, not tested. | | | |

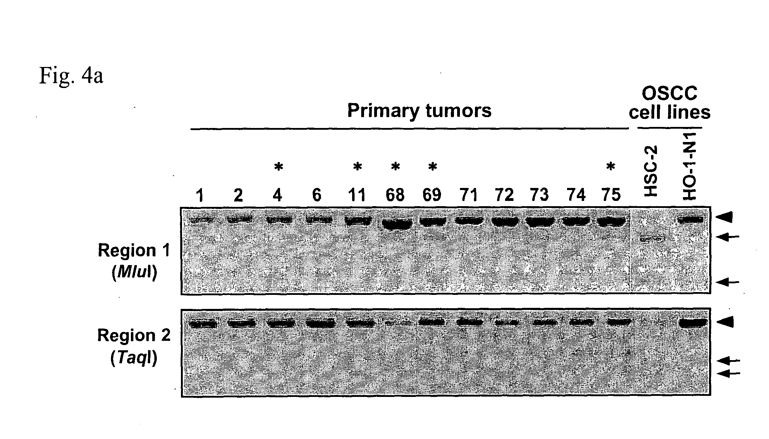

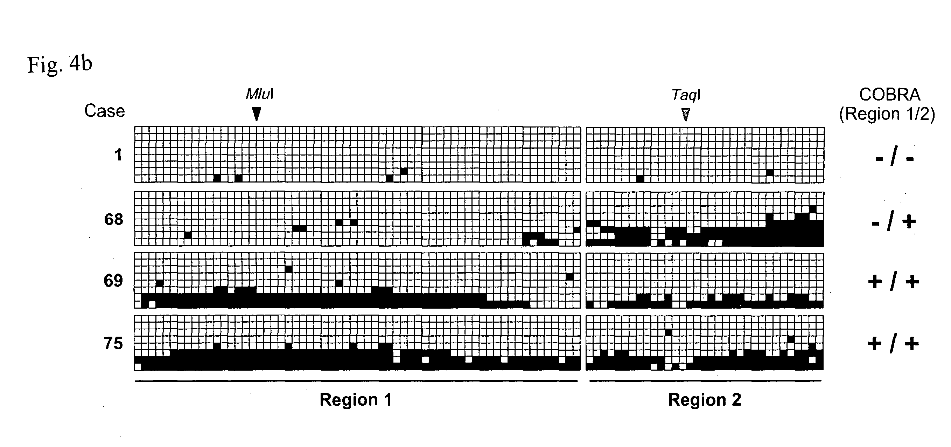

Next, in order to confirm the methylation status of the PRTFDC1 CpG island in oral squamous-cell carcinoma clinical specimens, 47 cases of oral squamous-cell carcinoma were subjected to analysis using a COBRA method (Fig. 4a). As a result, the methylated allele in the PRTFDC1 CpG island was confirmed in 8 out of 47 cases (17.0%). Confirmation of the unmethylated allele in all samples may be due to contaminated normal cells. Oral squamous-cell carcinoma cases determined by COBRA analysis to be positive (1, 68, 69, and 75, Fig. 4b) were subjected to confirmation of methylation status by bisulfite sequencing. As a result, the methylated allele could be confirmed in the COBRA-positive oral squamous-cell carcinoma cases.

Furthermore, Table 5 shows the results of examining the relation between methylation of the PRTFDC1 CpG island and clinicopathological diagnosis in the 47 cases of oral squamous-cell carcinoma.

**Table 5:**

| Table 3. Relation between clinicopathological data and hypermethylation of the *PRTFDC1* CpG island | | | | |
|---|---|---|---|---|
| | | n | *PRTFDC1* hypermethylation^{a} | *P*^{b} |
| Total | | 47 | 8(17.0%) | |
| Gender | | | | |
| | Male | 19 | 2 (10.5%) | 0.4448 |
| | Female | 28 | 6 (21.4%) | |
| Age | | | | |
| | median (range), yrs. | 69.0(49-90) | | |
| | >60 | 12 | 1 (8.3%) | 0.6593 |
| | ≤60 | 35 | 7 (20.0%) | |
| Smoking^{c} | | | | |
| | Smoker | 13 | 0 (0%) | 0.1317 |
| | Nonsmoker | 21 | 5 (23.8%) | |
| Alcohol^{d} | | | | |
| | Drinker | 10 | 0 (0%) | 0.1472 |
| | Nondrinker | 21 | 5 (23.8%) | |
| Differentiation^{e} | | | | |
| | well-mod | 34 | 6 (17.6%) | > 0.9999 |
| | poor | 4 | 1(25-0%) | |
| TNM classification | | | | |
| T stage | | | | |
| | T1 | 3 | 0(0%) | > 0.9999 |
| | T2-4 | 44 | 8(18.2%) | |
| N stage | | | | |
| | N0 | 32 | 4(12.5%) | 0.2455 |
| | N1-3 | 15 | 4(26.7%) | |
| M | stage | | | |
| | MD | 40 | 7 (17.5%) | > 0.9999 |
| | M1 | 7 | 1(14.3%) | |
| Stage | | | | |
| | I,II | 16 | 3(18.8%) | 0.6645 |
| | III, IV | 31 | 5(16.1%) | |

| | | | | |
|---|---|---|---|---|
| ^{a}Methylation status was determined by methylation-specific PCR as described in Materials and Methods. ^{b}*P* values are from the x2 or Fisher exact test and were statistically significant when < 0.05 (2-sided). ^{c}No information in 13 cases. ^{d}No information in 16 cases. ^{e}No information in 9 cases. | | | | |

### Example 7: Suppression of the proliferation of oral squamous-cell carcinoma by the activation of the PRTFDC1 gene

It was examined based on these results whether activation of PRTFDC1 gene expression resulted in suppressed proliferation of oral squamous-cell carcinoma. First, a plasmid (pCMV-3Tag4A-PRTFDC1) expressing the Myc tag of the PRTFDC1 gene was constructed. This can be used for monitoring the role of the full-length PRTFDC1 gene. This plasmid was prepared by inserting PRTFDC1 cDNA amplified by RT-PCR into a pCMV-3Tag4A vector (Stratagene) so that the Myc tag matched the translation frame. An empty vector (pCMV-3Tag4A-mock) in which no PRTFDC1 gene had been inserted was used as a control. These expression plasmids were mixed with a transfection reagent, FuGENE6 (Roch Diagnostics). NA and OM-2 cells were transfected with the plasmids. Cells were collected after 48 hours and then subjected to Western blot analysis using an anti-Myc antibody (Cell Signaling Technology). Thus, PRTFDC1 protein expression was confirmed (Fig. 5a).

On week 3 after transfection, cells that had proliferated in the presence of a neomycin-based drug, G418, were fixed with 70% ethanol, stained with crystal violet, and then counted. As a result, the colony count of the cells transfected with pCMV-3Tag4A-PRTFDC1 significantly decreased compared with that of the cells transfected with the empty vector (Fig. 5b). The results clearly demonstrate that the proliferation of oral squamous-cell carcinoma can be suppressed through activation of PRTFDC 1 gene expression and that PRTFDC 1 can function as an anticancer agent.

### Effect of the Invention

According to the present invention, it becomes possible to precisely understand signs of canceration and malignancy in the oral-cavity-derived cell specimen. Furthermore, proliferation of oral squamous-cell carcinoma can be suppressed by introducing a transcript of the PRTFDC1 gene that inactivates the gene expression in oral squamous-cell carcinoma. Furthermore, a therapeutic agent for oral squamous-cell carcinoma that is developed by inactivation of PRTFDC1 gene expression can be screened.

## Claims

1. A method for detecting cancer, which comprises detecting canceration including malignancy of a specimen through detection of at least one alteration of a gene existing in a chromosomal region 1q21, 2q24.1-q24.2, 3p13, 7p11.2, 10p12.1, 11p15.4, 11p15.2, 11p13.3, 11q22, 11q23.3, 12p13, 12q24.31, 13q33.3-q34, 12q24.1, 19q13, or 22q12.1 in the specimen.

2. The method for detecting cancer according to claim 1, wherein the gene is at least one of BCL9, MITF, EGFR, PTH, BCL1, FGF4, CCND1, FGF3, PGR, YAP1, CIAP1, MMP7, MMP1, CCND2, FGF6, BCL7A, DP1, GAS6, SUPT5H, PRTFDC1, c10orf63, THNSL1, GPR158, MY03A, or GAF2.

3. The method for detecting cancer according to claim 1, wherein the gene alteration is at least one of amplification, deletion, and inactivation.

4. The method for detecting cancer according to claim 1, wherein the gene alteration is inactivation due to methylation of a CpG island.

5. A method for detecting cancer, which comprises detecting canceration including malignancy of a specimen through detection of amplification of BCL9, MITF, EGFR, PTH, BCL1, FGF4, CCND1, FGF3, PGR, YAP1, CIAP1, MMP7, MMP1, CCND2, FGF6, BCL7A, DP1, GAS6, or SUPT5H gene or through detection of deletion of PRTFDC1, c10orf63, THNSL1, GPR158, MY03A, or GAF2 gene.

6. A method for detecting cancer, which comprises detecting canceration including malignancy of a specimen through detection of deletion or inactivation of a PRTFDC 1 gene.

7. The method for detecting cancer according to claim 1, wherein the specimen is tissue derived from the oral cavity.

8. The method for detecting cancer according to claim 1, wherein the cancer is oral squamous-cell carcinoma.

9. The method for detecting cancer according to claim 1, wherein the gene alteration is detected using a DNA chip method, a Southern blot method, a Northern blot method, a real-time RT-PCR method, a FISH method, a CGH method, an array CGH method, a bisulfite sequencing method, or a COBRA method.

10. A method for inhibiting cell growth, which comprises introducing a PRTFDC1, c10orf63, THNSL1, GPR158, MYO3A, or GAF2 gene, or a protein that is an expression product thereof into cells *in vitro.*

11. A cell growth inhibitor, which comprises a PRTFDC1, c10orf63, THNSL1, GPR158, MY03A, or GAF2 gene, or a protein that is an expression product thereof.

12. A method for activating cell growth, which comprises introducing siRNA, shRNA, an antisense oligonucleotide, or a loss-of-function type gene of a PRTFDC1, c10orf63, THNSL1, GPR158, MY03A, or GAF2 gene into tumor cells *in vitro.*

13. A cell growth activating agent, which comprises siRNA, shRNA, an antisense oligonucleotide, or a loss-of-function type gene of a PRTFDC1, c10orf63, THNSL1, GPR158, MYO3A, or GAF2 gene.

14. A method for screening for a substance, which comprises causing a test substance to come into contact with oral squamous-cell carcinoma in which gene expression is suppressed due to methylation of a CpG island of a PRTFDC1, c10orf63, THNSL1, GPR158, MYO3A, or GAF2 gene, detecting the expression of the gene, and then selecting a test substance as an anti-tumor substance capable of activating the relevant gene by demethylation of the CpG island of the gene when the gene expression is increased to a level higher than that in a system in which the test substance is not caused to come into contact therewith.


SEQUENCE LISTING
<110> FUJIFILM Corporation
<120> Method for Detecting Oral Squamous-cell Carcinoma and Method for
Suppressing the same
<130> A71053A
<160> 36
<210> 1
<211> 21
<212> DNA
<213> Artificial Sequence
<220>
<223> Description of Artificial Sequence: Synthetic DNA
<400> 1
caccttggct tcaacaaaca g                21
<210> 2
<211> 22
<212> DNA
<213> Artificial Sequence
<220>
<223> Description of Artificial Sequence: Synthetic DNA
<400> 2
ctggccaagg atattatgaa ag                22
<210> 3
<211> 22
<212> DNA
<213> Artificial Sequence
<220>
<223> Description of Artificial Sequence: Synthetic DNA

<400> 3

tataccaaag aagatccgca ag                22

<210> 4

<211> 21

<212> DNA

<213> Artificial Sequence

<220>

<223> Description of Artificial Sequence: Synthetic DNA

<400> 4

atgcattacc tccactgcat g                21

<210> 5

<211> 23

<212> DNA

<213> Artificial Sequence

<220>

<223> Description of Artificial Sequence: Synthetic DNA

<400> 5

atattgctac agaagcatat gag                23

<210> 6

<211> 20

<212> DNA

<213> Artificial Sequence

<220>

<223> Description of Artificial Sequence: Synthetic DNA

<400> 6

tttgcccagt cgttctggac                20

<210> 7

<211> 22

<212> DNA
<213> Artificial Sequence
<220>
<223> Description of Artificial Sequence: Synthetic DNA
<400> 7
tccagtgatt aaagccagaa tg                22
<210> 8
<211> 20
<212> DNA
<213> Artificial Sequence
<220>
<223> Description of Artificial Sequence: Synthetic DNA
<400> 8
caagaggcaa gagaacccag                20
<210> 9
<211> 21
<212> DNA
<213> Artificial Sequence
<220>
<223> Description of Artificial Sequence: Synthetic DNA
<400> 9
aacgtgtcag tggtggacct g                21
<210> 10
<211> 21
<212> DNA
<213> Artificial Sequence
<220>
<223> Description of Artificial Sequence: Synthetic DNA

<400> 10

gttggtgaag agaacatcca g                    21

<210> 11

<211> 22

<212> DNA

<213> Artificial Sequence

<220>

<223> Description of Artificial Sequence: Synthetic DNA

<400> 11

tataccaaag aagatccgca ag                    22

<210> 12

<211> 20

<212> DNA

<213> Artificial Sequence

<220>

<223> Description of Artificial Sequence: Synthetic DNA

<400> 12

attacctccc ttgacggctc                    20

<210> 13

<211> 23

<212> DNA

<213> Artificial Sequence

<220>

<223> Description of Artificial Sequence: Synthetic DNA

<400> 13

atattgctac agaagcatat gag                    23

<210> 14

<211> 20

<212> DNA
<213> Artificial Sequence
<220>
<223> Description of Artificial Sequence: Synthetic DNA
<400> 14
gagaagcatg tggtttggca                    20
<210> 15
<211> 20
<212> DNA
<213> Artificial Sequence
<220>
<223> Description of Artificial Sequence: Synthetic DNA
<400> 15
agagagaatg agtcgcctct                    20
<210> 16
<211> 22
<212> DNA
<213> Artificial Sequence
<220>
<223> Description of Artificial Sequence: Synthetic DNA
<400> 16
aattagattt tggtggtatt gg                 22
<210> 17
<211> 21
<212> DNA
<213> Artificial Sequence
<220>
<223> Description of Artificial Sequence: Synthetic DNA

<400> 17

tggatttggg aattaggggt t                21

<210> 18

<211> 25

<212> DNA

<213> Artificial Sequence

<220>

<223> Description of Artificial Sequence: Synthetic DNA

<400> 18

aaatgtaaat tttttatttt ggggt                25

<210> 19

<211> 19

<212> DNA

<213> Artificial Sequence

<220>

<223> Description of Artificial Sequence: Synthetic DNA

<400> 19

ggttgggcat tcgctgcag                19

<210> 20

<211> 22

<212> DNA

<213> Artificial Sequence

<220>

<223> Description of Artificial Sequence: Synthetic DNA

<400> 20

ccttcattga gacaaatcga tc                22

<210> 21

<211> 25

<212> DNA

<213> Artificial Sequence

<220>

<223> Description of Artificial Sequence: Synthetic DNA

<400> 21

gtgttctaat aaatataacg gttat                25

<210> 22

<211> 22

<212> DNA

<213> Artificial Sequence

<220>

<223> Description of Artificial Sequence: Synthetic DNA

<400> 22

gattttggac aagttgttcc ac                22

<210> 23

<211> 20

<212> DNA

<213> Artificial Sequence

<220>

<223> Description of Artificial Sequence: Synthetic DNA

<400> 23

tcctctggat ccaagctgtg                20

<210> 24

<211> 24

<212> DNA

<213> Artificial Sequence

<220>

<223> Description of Artificial Sequence: Synthetic DNA

<400> 24

tttacctttc attagcctta atac                24

<210> 25

<211> 20

<212> DNA

<213> Artificial Sequence

<220>

<223> Description of Artificial Sequence: Synthetic DNA

<400> 25

ttgccgctgg gtttgagatg                20

<210> 26

<211> 20

<212> DNA

<213> Artificial Sequence

<220>

<223> Description of Artificial Sequence: Synthetic DNA

<400> 26

aggacacgtt gcctctcttc                20

<210> 27

<211> 20

<212> DNA

<213> Artificial Sequence

<220>

<223> Description of Artificial Sequence: Synthetic DNA

<400> 27

agtgggtgtc gctgttgaag                20

<210> 28

<211> 23

<212> DNA
<213> Artificial Sequence
<220>
<223> Description of Artificial Sequence: Synthetic DNA
<400> 28
tcagatctct gaagtattca ttg 23
<210> 29
<211> 23
<212> DNA
<213> Artificial Sequence
<220>
<223> Description of Artificial Sequence: Synthetic DNA
<400> 29
aaagttgtgc tgttggtatc atg 23
<210> 30
<211> 22
<212> DNA
<213> Artificial Sequence
<220>
<223> Description of Artificial Sequence: Synthetic DNA
<400> 30
gaaatcgctg tgcatgttta tc 22
<210> 31
<211> 22
<212> DNA
<213> Artificial Sequence
<220>
<223> Description of Artificial Sequence: Synthetic DNA

<400> 31

atatttccag ttgggcatag ag                    22

<210> 321

<211> 20

<212> DNA

<213> Artificial Sequence

<220>

<223> Description of Artificial Sequence: Synthetic DNA

<400> 32

attggcttat ggtgcgagac                    20

<210> 33

<211> 20

<212> DNA

<213> Artificial Sequence

<220>

<223> Description of Artificial Sequence: Synthetic DNA

<400> 33

ttgccgctgg gtttgagatg                    20

<210> 34

<211> 19

<212> DNA

<213> Artificial Sequence

<220>

<223> Description of Artificial Sequence: Synthetic DNA

<400> 34

accctttccc ctctcctac                    19

<210> 35

<211> 25

<212> DNA

<213> Artificial Sequence

<220>

<223> Description of Artificial Sequence: Synthetic DNA

<400> 35

accccaaaat aaaaaattta cattt 25

<210> 36

<211> 23

<212> DNA

<213> Artificial Sequence

<220>

<223> Description of Artificial Sequence: Synthetic DNA

<400> 36

cccaaaaaaa aaaaaacctc caa 23